# EUROPÄISCHE PATENTSCHRIFT

(11) **EP 3 801 439 B1**
(45) Veröffentlichungstag und Bekanntmachung des Hinweises auf die Patenterteilung: **04.01.2023**
(21) Anmeldenummer: 19725739.7
(22) Anmeldetag: 24.05.2019
(51) Int. Cl.: A61J 9/00, A61M 1/06

(54) **BABYFLASCHE MIT MILCHPUMPE**
BABY BOTTLE WITH BREAST PUMP
BIBERON À POMPE À LAIT

(30) Priorität: 25.05.2018 EP 18174358
(43) Veröffentlichungstag der Anmeldung: 14.04.2021
(73) Patentinhaber: Medela Holding AG, 6340 Baar (CH)
(72) Erfinder: FELBER, Armin, 6003 Luzern (CH); THÜRING, Martin, 5643 Sins (CH); HÖNER, Sebastian, 8800 Thalwil (CH)
(74) Vertreter: Grünecker Patent- und Rechtsanwälte PartG mbB
(86) Internationale Anmeldenummer: PCT/EP2019/063500
(87) Internationale Veröffentlichungsnummer: WO 2019/224365

(56) Entgegenhaltungen:
- WO-A1-2016/014469
- CN-A- 105 267 044
- CN-A- 106 137 768
- CN-U- 206 730 161
- US-A1- 2010 012 395
- US-A1- 2015 283 311
- US-A1- 2017 021 068

## Beschreibung

Die vorliegende Erfindung betrifft eine Vorrichtung, die einen Konnektor, eine daran ankoppelbare Babyflasche mit einem Flaschenkörper und einem Flaschenboden, und eine in dem Flaschenboden angeordnete Sensorik zur Bestimmung der Milch-Füllmenge in der Babyflasche umfasst. Eine solche Vorrichtung ist aus der US 2015/0283311 A1 bekannt. Diese Vorrichtung kann auch als Pumpeinheit bezeichnet werden.

Die US 2015/028331 1 A1 beschreibt eine Vorrichtung umfassend eine ein Reservoir aufweisende Babyflasche, die an ihrem Flaschenhals mit einem Konnektor verschraubt ist. Bei aufrechtstehender Babyflasche bildet eine Platte die untere Begrenzung des Reservoirs und das Gewicht in dem Reservoir gesammelter Flüssigkeit lastet auf der Platte. Auf der gegenüberliegenden Seite der Platte ist ein Dehnungsmessstreifen angeordnet, der die Zu- oder Abnahme der Gewichtslast auf die Platte registriert. Anhand dieser Messung kann die Füllmenge der Babyflasche berechnet werden.

Die CN 105 267 044 A gibt eine Vorrichtung mit den oberbegrifflichen Merkmalen von Anspruch 1 und eine Flasche mit den oberbegrifflichen Merkmalen von Anspruch 6 an.

Die vorbekannten Vorrichtungen lassen Raum zur Verbesserung.

Eine Aufgabe der vorliegenden Erfindung ist es, die Handhabung und Wartung der Vorrichtung, sowie die Bestimmung der Inhaltsmenge zu verbessern.

Zur Lösung dieser Aufgabenstellung schlägt die vorliegende Erfindung eine Vorrichtung mit den Merkmalen von Anspruch 1 vor. Der Flaschenboden dieser Vorrichtung ist lösbar am Flaschenkörper befestigt.

So kann der Flaschenboden mit der darin angeordneten Sensorik von dem Flaschenkörper getrennt werden. Damit ist die Sensorik zu Wartungs- oder Reparaturzwecken besser zugänglich und der Flaschenkörper kann besser gereinigt werden. Des Weiteren wird das Design des Flaschenkörpers nicht durch die Sensorik beeinträchtigt.

Die erfindungsgemäße Babyflasche ist in der Regel aus einem zumindest teilweise transparenten thermoplastischen Kunststoff oder aus Glas hergestellt. Besonders bevorzugt wird Polypropylen verwendet. Die Babyflasche aus Kunststoff kann als Einwegflasche oder als Mehrwegflasche produziert sein und hat meist ein Gewicht von 7,5 bis 30 g. Die Mehrweg-Babyflasche hat vorzugsweise eine Wandstärke von ca. 0,9 mm.

Das Nennvolumen der Babyflasche beträgt üblicherweise 80 bis 250 ml, insbesondere 80 ml, 150 ml oder 250 ml. Das Maximalvolumen, das die Babyflasche aufnehmen kann, ist in der Regel nicht größer als 330 ml.

Die Babyflasche weist bevorzugt meist eine Höhe von ca. 60 bis 160 mm auf, vorzugsweise 66 mm, 99,5 mm, 102 mm, 136 mm oder 148,5 mm. Der Durchmesser des eine Öffnung ausbildenden Flaschenhalses ist in der Regel 33 mm. Üblicherweise ist der maximale Durchmesser des Flaschenkörpers nicht größer als 50 bis 70 mm, bevorzugt nicht größer als 53 mm, 60 mm oder 65 mm. Sämtliche Maße sind mit einer Toleranz von ± 10%, bevorzugt ± 5% zu verstehen.

In der Regel hat der Flaschenkörper einen im Wesentlichen zylindrischen Flaschenbauch, der sich zu einem Flaschenhals konisch verjüngt, wobei der Flaschenhals mit einer Milchpumpe und/oder einem Trinksauger verbunden werden kann. Der Durchmesser des Flaschenbauchs kann aber auch über seine Länge hinweg variieren. Beispielsweise kann der Flaschenkörper mehrere zylindrische Abschnitte unterschiedlichen Durchmessers aufweisen, die insbesondere über einen oder mehrere konisch geformte Abschnitte verbunden sein können. Der Flaschenhals hat üblicherweise den geringsten Durchmesser. Vorzugsweise ist der Flaschenhals mit einem Außengewinde versehen.

Der Flaschenboden bildet für gewöhnlich eine Standfläche aus, auf der die Babyflasche auf einer ebenen Auflagefläche aufrecht steht. Üblicherweise wird der Teil der Babyflasche als Flaschenboden bezeichnet, der sich von der Standfläche bis zu einem flüssigkeits-undurchlässigen Trennelement erstreckt, welches ein Flüssigkeitsreservoir der Babyflasche in Richtung der Standfläche begrenzt. Das Trennelement kann dem Flaschenkörper oder dem Flaschenboden zugeordnet sein. Der abnehmbare Flaschenboden ist in der Regel scheibenförmig mit einer Höhe von ca. 1 bis 3 cm. Der Durchmesser des abnehmbaren Flaschenkörpers entspricht meist dem des bodenseitigen Endes des Flaschenkörpers, kann aber bis zu 20 mm davon abweichen. Die Standfläche ist für gewöhnlich eine im Wesentlichen ebene Fläche, die ggf. eine zentrale Wölbung nach innen in Richtung des Reservoirs aufweisen kann. Die Standfläche kann auch durch einen nach unten vorstehenden Ring gebildet sein.

Ein Konnektor umfasst in der Regel eine an die Form einer menschlichen weiblichen Brust angepasste und insbesondere dichtend daran anlegbare Brusthaube, die mit einer Handpumpe oder einer elektrisch betriebenen Pumpe verbindbar ist und zusammenwirkt, um einen Unterdruck zwischen der Brusthaube und der Brust zu erzeugen. Der Unterdruck wird üblicherweise in einer bestimmten Frequenz entsprechend den Pump- bzw. Saughüben der Pumpe erzeugt. Dadurch wird der Milchfluss stimuliert. Der Konnektor weist meist einen Kanal auf, der in der Brusthaube aufgefangene Milch in die Babyflasche abführt, sofern der Konnektor mit der Babyflasche verbunden ist. Üblicherweise ist in dem Kanal ein Klappventil vorgesehen, welches im Pump- bzw. Saughub geschlossen bleibt. Im Gegenhub öffnet sich das Klappventil und Milch fließt hindurch. Bevorzugt beinhaltet die Vorrichtung einen Trichter, der geeignet ausgebildet ist, um Milch zentral in das untere Ende, d.h. das standflächenseitige Ende, des Reservoirs einzuleiten.

Die Vorrichtung hat ein flüssigkeits-undurchlässiges Trennelement, das als elastische Membran ausgebildet ist, die in Richtung der Sensorik beweglich ist und einen zentrisch angeordneten metallischen Einsatz oder eine metallische Beschichtung als Messelement aufweist. Die Membran kann randseitig an der Babyflasche fixiert sein und weist eine Beweglichkeit im Sinne einer elastischen Verformung auf. Ohne Krafteinwirkung der Gewichtskraft einer Milch-Füllmenge, d. h. bei leerer Babyflasche, kehrt die Membran in ihre Ausgangsposition zurück bzw. verbleibt dort. Die Membran ist bevorzugt als separates Bauteil ausgebildet, das durch Lösen des Flaschenbodens vom Flaschenkörper leicht entfernt und gereinigt bzw. sterilisiert werden kann. Insbesondere ist die Membran so in der Babyflasche positionierbar, dass es das Reservoir in Richtung des Flaschenbodens fluiddicht abschließt und den Flaschenboden und den Flaschenkörperfluiddicht voneinander trennt. Der metallische Einsatz oder die metallische Beschichtung ist in der Regel auf der dem Reservoir gegenüberliegenden Seite der Membran angeordnet. So ist sowohl die Sensorik als auch das Messelement von der Milch in der Babyflasche getrennt, sodass eine störungsfreie Messung gewährleistet werden kann.

Nach einer bevorzugten Weiterbildung der vorliegenden Erfindung ist die Membran derart ausgebildet, dass sie sich - bei aufrecht stehender Babyflasche - bei Zunahme der Milch-Füllmenge auf die Sensorik hin und bei Abnahme der Milch-Füllmenge von der Sensorik weg bewegt. Der Abstand des Messeelements von der Sensorik verringert sich mit zunehmender Milch-Füllmenge in der Babyflasche. Eine sensible Messung dieses Abstands ermöglicht eine besonders exakte Ermittlung der Milch-Füllmenge in der Babyflasche.

Die Sensorik weist eine Spule auf, die zentral im Flaschenboden und zu der Membran beabstandet angeordnet ist.

Dabei wird das Magnetfeld der Spule durch in dem Messelement induzierte Wirbelströme beeinflusst, wobei dieser Einfluss proportional zu dem Abstand zwischen dem metallischen Einsatz bzw. der metallischen Beschichtung und der Spule ist. Die Stärke des Einflusses und dessen zeitliche Veränderung auf das Magnetfeld der Spule lassen sich beispielsweise mithilfe eines RLC-Schwingkreises erfassen, welcher ebenfalls in dem Flaschenboden angeordnet sein kann. Bevorzugt verbleibt auch bei maximal gefüllter Babyflasche ein minimaler Abstand bzw. Spalt zwischen dem Messelement und der Spule von ungefähr 1 mm.

Der RLC-Schwingkreis ist bevorzugt auf einer Leiterplatte (PCB) vorgesehen. Dies erweist sich als eine platzsparende und kostengünstige Lösung, die Magnetfeldänderungen der Spule zu erfassen.

Nach einer bevorzugten Weiterbildung der vorliegenden Erfindung fluchtet die Achse des metallischen Einsatzes mit der Achse der Spule zumindest angenähert. Besonders bevorzugt ist die Achse des metallischen Einsatzes koaxial mit der Achse der Spule angeordnet. Dies erhöht die Messgenauigkeit. Als Achse des metallischen Einsatzes ist insbesondere die Längsachse bzw. im Falle eines radialsymmetrischen Einsatzes die Symmetrieachse zu verstehen. Als metallischer Einsatz eignet sich beispielsweise eine bevorzugt scheibenförmige Kupferplatte oder eine Platte aus rostfreiem Stahl oder aus eloxiertem Aluminium.

Nach einer weiteren bevorzugten Weiterbildung der vorliegenden Erfindung sind zwischen der Spule und der durch den Flaschenboden ausgebildeten Standfläche elektronische Bauteile angeordnet. Insbesondere bilden die elektronischen Bauteile zusammen mit der Spule den RLC-Schwingkreis. Es können jedoch weitere elektronische Bauteile wie beispielsweise ein Mikroprozessor oder ein Lagesensor vorgesehen sein. Besonders bevorzugt sind die elektronischen Bauteile auf einer Leiterplatte (PCB) vorgesehen, auf der auch die Spule angeordnet ist. Die Leiterplatte und die Spule weisen vorzugsweise ein zentrales Loch auf. Hiermit kann ein Luftdruck ausgeglichen werden, der durch die Bewegung der Membran in Richtung der Sensorik verursacht wird.

Vorzugsweise ist der Flaschenboden durch Verschrauben, einen Bajonettverschluss oder einen Schnappverschluss an der Babyflasche befestigbar. Bevorzugt hat im Falle eines Bajonettverschluss der Flaschenboden zumindest einen eine Hinterschneidung aufweisenden Arretierarm und der Flaschenkörper zumindest einen zu der Hinterschneidung parallelen Arretiersteg an seinem Innenumfang. Im Falle eines Verschraubens ist es der kompakten Ausgestaltung wegen zu bevorzugen ein Innengewinde an der Innenumfangsfläche des Flaschenkörpers und ein Gegengewinde am Flaschenboden vorzusehen. Genauso gut kann aber der Flaschenboden ein Innengewinde und der Flaschenköper ein Außengewinde als Gegengewinde ausbilden. Im Falle eines Schnappverschluss weist der Flaschenboden bevorzugt zumindest eine Rastnase auf, die durch manuelle Betätigung elastisch rückgehalten werden kann, um sie bei dem Zusammenführen von Flaschenboden und Flaschenkörper hinter einen am Flaschenkörper ausgebildeten Rastvorsprung einzurasten. Der Rastvorsprung ist bevorzugt als umfänglich durchgängiger Steg an der Innenumfangsfläche des dem Flaschenboden zugewandten Endes des Flaschenkörpers ausgebildet.

Bevorzugt weist der Flaschenkörper einen ringförmigen Flansch an der Innenumfangsfläche des dem Flaschenboden zugewandten Endabschnitts des Flaschenkörpers auf, gegen den der Flaschenboden bzw. die Membran dichtend anliegt. Weiter bevorzugt wird die Membran beim Verbinden des Flaschenköpers mit dem Flaschenboden gegen den Flansch gepresst und fixiert.

In einem nebengeordneten Aspekt gibt die vorliegende Erfindung eine Flasche mit den Merkmalen von Anspruch 6 an.

Weitere Einzelheiten der vorliegenden Erfindung ergeben sich aus der nachfolgenden Beschreibung eines Ausführungsbeispiels der Erfindung in Verbindung mit der Zeichnung. In dieser zeigen:
- Fig. 1A: eine Seitenansicht eines Ausführungsbeispiels,
- Fig. 1B: eine Seitenansicht des mit Milch gefüllten Ausführungsbeispiels aus Figur 1A,
- Fig. 2A: eine Längsschnittansicht eines zweiten Ausführungsbeispiels,
- Fig. 2B: eine Explosionsdarstellung des zweiten Ausführungsbeispiels aus Figur 2A (ohne Konnektor),
- Fig. 3A-3D: eine vergrößerte Ansicht des Flaschenbodens des ersten und des zweiten Ausführungsbeispiels in Explosionsdarstellung (3A), perspektivischer Seitenansicht (3B), Schnittansicht (3C) und Seitenansicht (3D),
- Fig. 4A-4C: eine vergrößerte Ansicht der Membran des ersten und des zweiten Ausführungsbeispiels mit Blick von oben (4A), mit Blick von unten (4B) und in Längsschnittansicht (4C),
- Fig. 5A, 5B: eine Draufsicht auf die Oberseite (5A) und die Unterseite (5B) der Leiterplatte des ersten und des zweiten Ausführungsbeispiels mit Sensorik,
- Fig. 6A, 6B: eine perspektivische Seitenansicht des ersten Ausführungsbeispiels (ohne Konnektor),
- Fig. 7A, 7B: eine perspektivische Seitenansicht eines dritten Ausführungsbeispiels,
- Fig. 8A, 8B: eine perspektivische Seitenansicht eines vierten Ausführungsbeispiels,
- Fig. 9A, 9B: eine perspektivische Seitenansicht eines fünften Ausführungsbeispiels,
- Fig. 10: ein Schaubild über den Zusammenhang zwischen dem Abstand der Membran von der Spule und der Milch-Füllmenge, und
- Fig. 11: ein Schaubild über den Zusammenhang zwischen der Milch-Füllmenge und der Ausgangsfrequenz eines RLC-Schwingkreise.

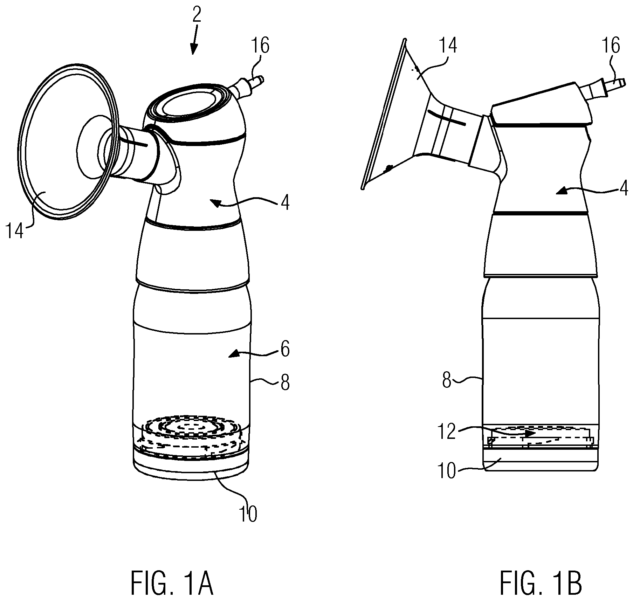

Figur 1A zeigt ein Ausführungsbeispiel der erfindungsgemäßen Vorrichtung 2. Die Vorrichtung 2 ist eine Pumpeinheit und weist einen Konnektor 4 auf, der über eine nicht dargestellte Schraubverbindung an einer Babyflasche 6 befestigt ist. Die Babyflasche 6 hat einen im Wesentlichen zylindrischen Flaschenkörper 8 und einen Flaschenboden 10. Der Flaschenkörper 8 ist in dem vorliegenden Ausführungsbeispiel aus transparentem Polypropylen hergestellt. Man erkennt daher ein flüssigkeitsundurchlässiges Trennelement 12, welches in der Babyflasche 6 angeordnet ist. Das flüssigkeitsundurchlässige Trennelement 12 dichtet ein Reservoir des Flaschenkörpers 8 fluiddicht gegenüber dem Flaschenboden 10 ab. Dies ist in Figur 1B verdeutlicht, in der die mit Milch gefüllte Babyflasche dargestellt ist. In den Figuren 1A und 1B ist der Flaschenboden 10 mit dem Flaschenkörper 8 verbunden. Diese Verbindung ist lösbar.

Der Konnektor 4 weist eine Brusthaube 14, die an eine weibliche Brust dichtend anlegbar ist, und einen Anschluss 16 für eine Brustpumpe auf.

Gleiche Bauteile sind in den nachfolgend beschriebenen Ausführungsbeispielen mit den gleichen Bezugszeichen versehen.

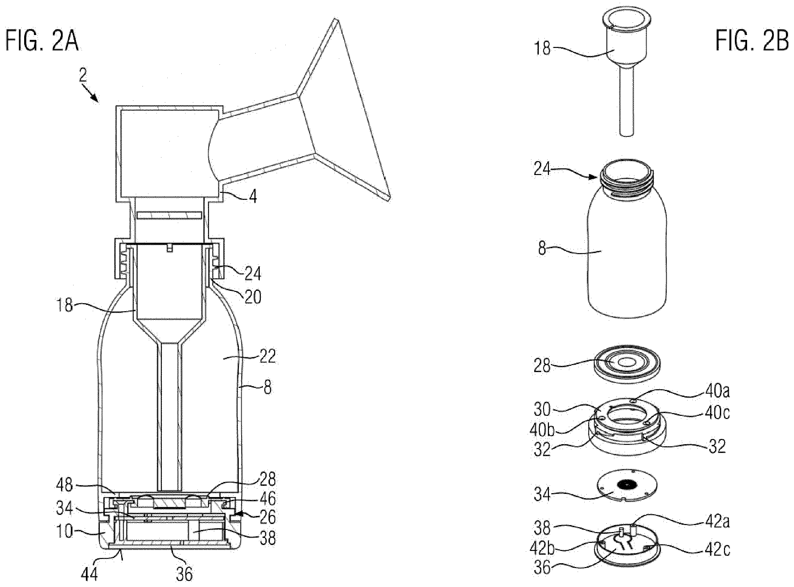

Figur 2A zeigt eine Schnittansicht eines zweiten Ausführungsbeispiels der erfindungsgemäßen Vorrichtung 2. Die Vorrichtung weist gegenüber dem Ausführungsbeispiel aus der Figur 1 zusätzlich einen Trichter 18 auf, welcher in den Flaschenhals 20 des Flaschenkörpers 8 eingesteckt ist und sich bis unmittelbar kurz vor dem bodenseitigen Ende des Flüssigkeitsreservoirs 22 der Babyflasche 8 erstreckt. Der Trichter 18 ist zentriert in der Babyflasche angeordnet.

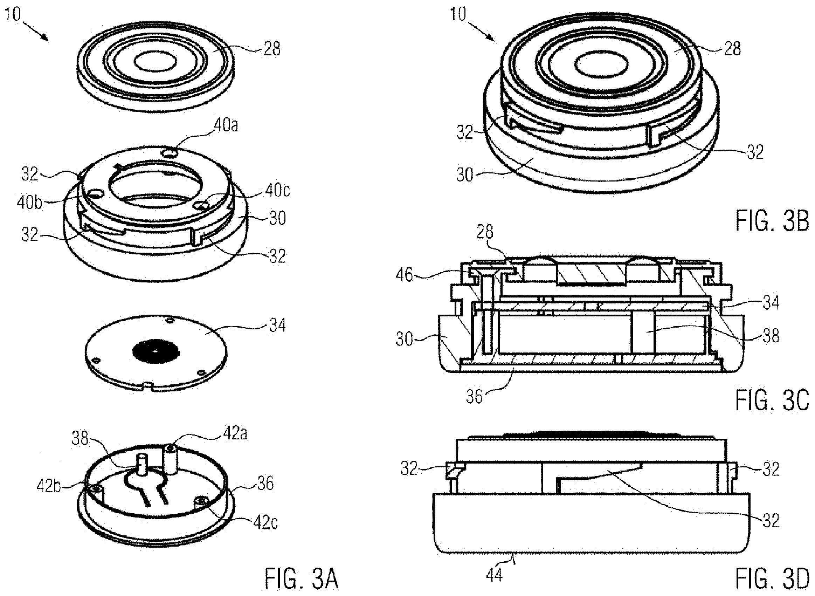

Die Figur 2B zeigt eine Explosionsdarstellung des Ausführungsbeispiels aus Figur 2A, wobei der Konnektor, der ein herkömmlicher sein kann, weggelassen wurde. Der Flaschenkörper 8 weist an dem Flaschenhals 20 ein Außengewinde 24 auf, über das der Konnektor 4 mit dem Flaschenkörper 8 verschraubt ist. Der Flaschenboden 10 ist über einen Bajonettverschluss 26 an dem Flaschenkörper 8 befestigt. Als flüssigkeits-undurchlässiges Trennelement 12 ist eine im Wesentlichen scheibenförmige elastische Membran 28 vorgesehen, die von einem Gehäuse 30 getragen ist, welches Arretierarme 32 für den Bajonettverschluss 26 ausbildet (siehe auch Figur 3D). In dem Flaschenboden 10 ist eine Sensorik 34 angeordnet, die in Figur 2B als eine bestückte scheibenförmigem Leiterplatte dargestellt ist. Der Flaschenboden 10 weist des Weiteren einen Gehäusedeckel 36 mit einem Distanzüberbrückungsbolzen 38 auf, der einen Ein-/Aus-Schalter betätigt, der nachstehend noch näher erläutert ist. Über einander zugeordnete Befestigungslöcher 40a, 40b, 40c in dem Gehäuse 30 einerseits und 42a, 42b, 42c in dem Gehäusedeckel 36 andererseits ist der Gehäusedeckel 36 fest mit dem Gehäuse 30 verbunden, vorzugsweise verschraubt. Der Gehäusedeckel 36 bildet eine ebene Standfläche 44 aus, auf der die Babyflasche 6 aufrecht steht, sofern die Babyflasche 6 auf eine ebene Auflagefläche gestellt wird. Bei dem Ausführungsbeispiel wird die Babyflasche über eine ebene Standfläche abgestützt, die durch einen in Fig. 2A erkennbaren unteren Ring gebildet ist.

Der äußere Rand der Membran 28 ist über einen Gehäuseflansch 46 gestülpt, sodass die elastische Membran 28 formschlüssig mit dem Gehäuse 30 verbunden ist (siehe auch Figur 3C). Aufgrund ihrer Elastizität kann die Membran 28 jedoch, insbesondere manuell wieder von dem Gehäuseflansch 44 entfernt werden, nachdem der Flaschenboden 10 von dem Flaschenkörper 8 gelöst wurde. Die Oberseite des äußeren Rands der Membran 28 wird von dem Gehäuse 30 gegen einen ringförmigen Flansch 48 gepresst, der am unteren Endabschnitt des Flaschenkörpers 8 an dessen Innenumfang ausgebildet ist.

Die Figuren 3A bis 3D zeigen verschiedene vergrößerte Ansichten des Flaschenbodens 10 des ersten und des zweiten Ausführungsbeispiels. Die Figur 3A ist eine Explosionsdarstellung und bildet die elastische Membran 28, das Gehäuse 30, die Sensorik 34 und den Gehäusedeckel 36 ab. Figur 3B zeigt eine perspektivische Seitenansicht des aus den in Figur 3A gezeigten Bauteilen zusammengesetzten Flaschenbodens 10.

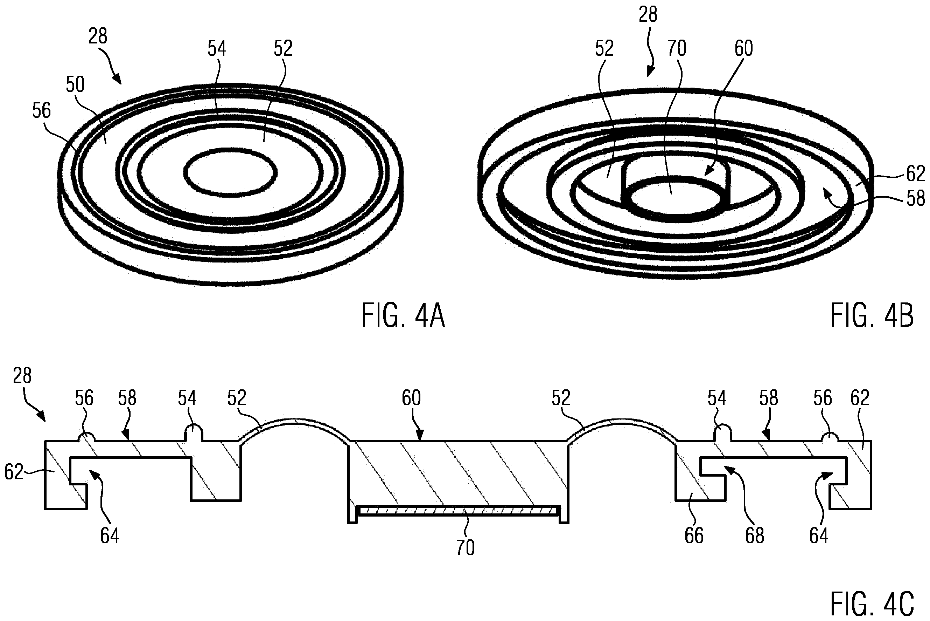

In den Figuren 4A, 4B und 4C ist die im Wesentlichen scheibenförmige elastische Membran 28 des ersten und des zweiten Ausführungsbeispiels vergrößerter dargestellt, wobei Fig. 4A die Oberseite, Fig. 4B die Unterseite und Fig. 4C eine Längsschnittansicht der Membran zeigt. Auf der im Wesentlichen ebenen Oberseite 50 der Membran 28 sind drei unterschiedliche Ringe ausgebildet, die aus der ebenen Oberfläche 50 hervorstehen: ein innerer bogenförmiger Ring 52, ein mittlerer Ring 54 und ein äußerer Ring 56. Im Bereich des bogenförmigen inneren Rings 52 weist die Membran 28 die geringste Dicke auf. Der bogenförmige innere Ring 52 verbindet einen äußeren Befestigungsabschnitt 58 mit einem inneren beweglichen Abschnitt 60. Während der äußere Befestigungsabschnitt 58 am Gehäuse 30 anliegt und über einen randseitigen, umfänglich geschlossenen Flansch 62 mit Hinterschneidung 64 über das Gehäuse 30 gestülpt und formschlüssig verbunden werden kann, ist der bogenförmige innere Ring 52 und der bewegliche Abschnitt 60 frei im Gehäuse 30 gehalten bzw. ist nicht vom Gehäuse 30 abgestützt.

Der äußere Befestigungsabschnitt 58 weist im vorliegenden Fall zumindest einen zweiten Flansch 66 mit Hinterschneidung 68 am radial inneren Ende des Befestigungsabschnitts 58 auf. Der bogenförmige innere Ring 52 ist elastisch verformbar und ermöglicht so die Beweglichkeit des beweglichen Abschnitts 60 orthogonal zu der ebenen Oberseite 50 der Membran 28. Auf seiner Unterseite weist der bewegliche innere Abschnitt 60 eine Kupferplatte 70 als metallischen Einsatz auf.

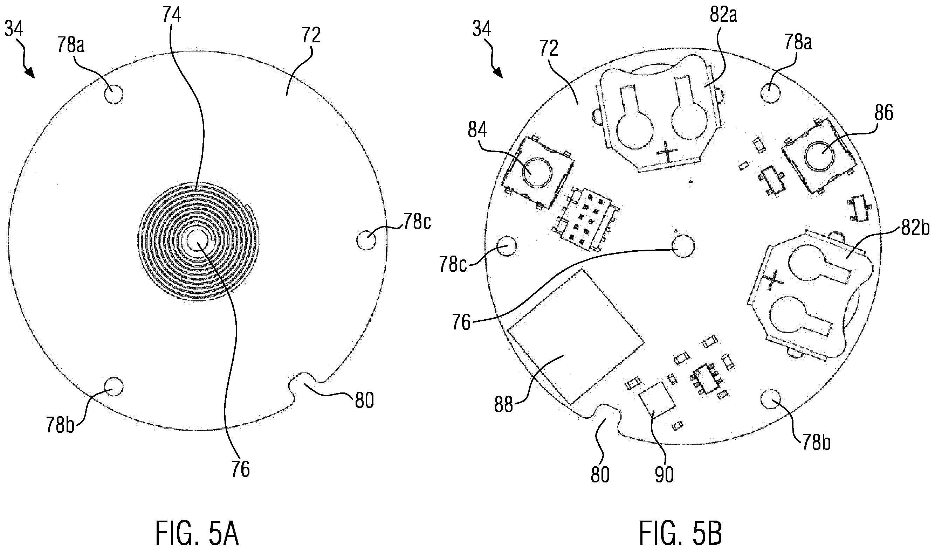

In den Figuren 5A und 5B ist die Sensorik 34 des ersten und des zweiten Ausführungsbeispiels dargestellt, die auf einer scheibenförmigen Leiterplatte 72 angeordnet ist. Fig. 5A zeigt die Oberseite der Leiterplatte 72, auf der eine Spule 74 angeordnet ist. In der Mitte der Leiterplatte ist ein Loch 76 für den Luftdruckausgleich vorgesehen. Am äußeren Randbereich der Leiterplatte 72 sind drei Durchgangslöcher 78a, 78b, 78c angeordnet, durch die jeweils ein Befestigungselement hindurch geführt werden kann. Der äußere Rand der Leiterplatte 72 weist zudem eine Kerbe 80 auf, die bei der Montage eine genaue Positionierung ermöglicht.

Die Figur 5B zeigt die Unterseite der Leiterplatte 72, die mit Batterien 82a, 82b einem Ein/AusSchalter 84, einem Reset-Schalter 86, einem Mikroprozessor 88 und einem Lagesensor 90 bestückt ist.

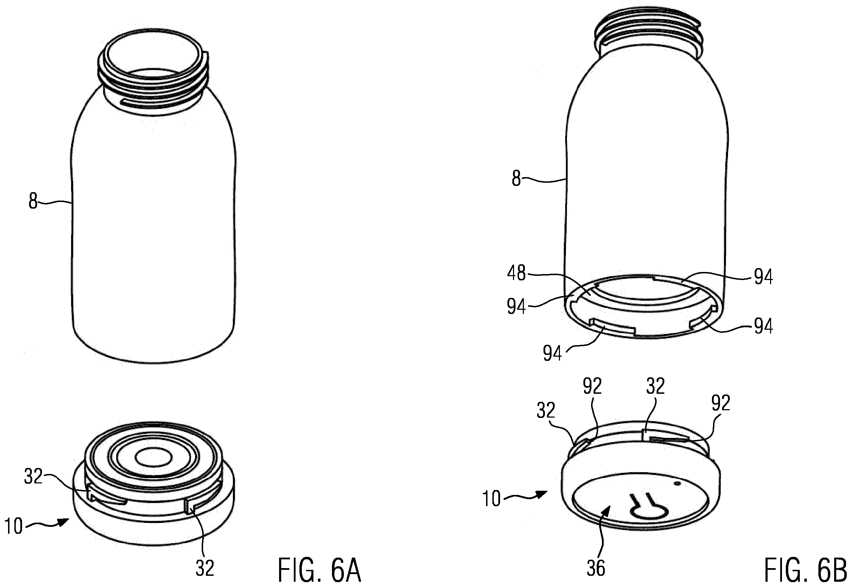

Die nachfolgend beschriebenen Figuren 6 bis 9 beziehen sich auf unterschiedliche Ausführungsbeispiele, die sich jedoch lediglich in dem Verbindungsmechanismus zum lösbaren Verbinden des Flaschenbodens 10 mit dem Flaschenkörper 8 unterscheiden. Dabei hat der Flaschenkörper 8 stets einen ringförmigen Flansch 48 an der Innenumfangsfläche des dem Flaschenboden zugewandten Endabschnitts des Flaschenkörpers 8, gegen den der Befestigungsabschnitt 58 der Membran 28 dichtend anliegt, wenn der Flaschenboden 10 an dem Flaschenkörper 8 befestigt ist. Die Figuren 6A, 6B beziehen sich auf das erste Ausführungsbeispiel welches einen Bajonettverschluss aufweist. Nach diesem Ausführungsbeispiel bildet das Gehäuse 30 des Flaschenbodens 10 Arretierarme 32 aus. Die Arretierarme 32 haben eine Hinterschneidung 92, in die sich durch eine Drehbewegung - nachdem der Flaschenboden in den Flaschenkörper eingeführt wurde - Arretierstege 94 des Flaschenkörpers einführen lassen.

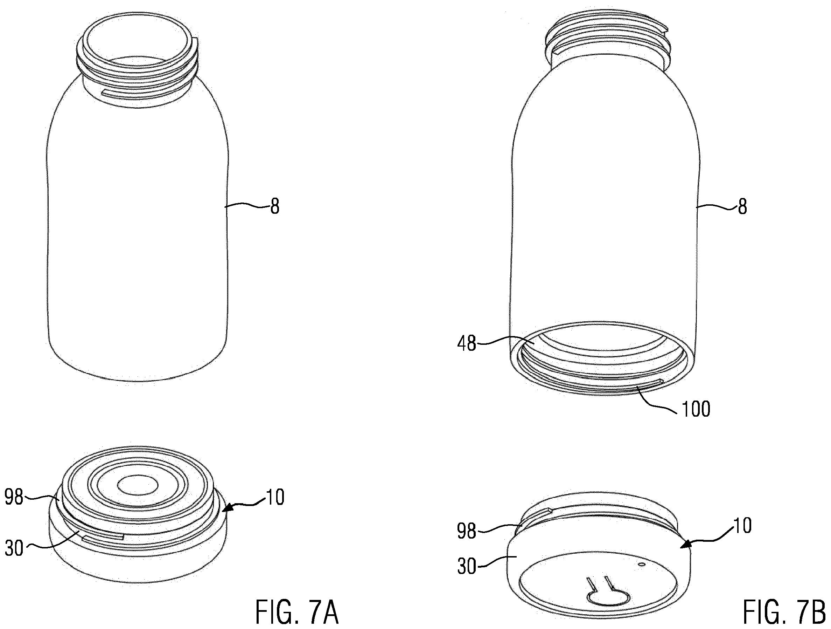

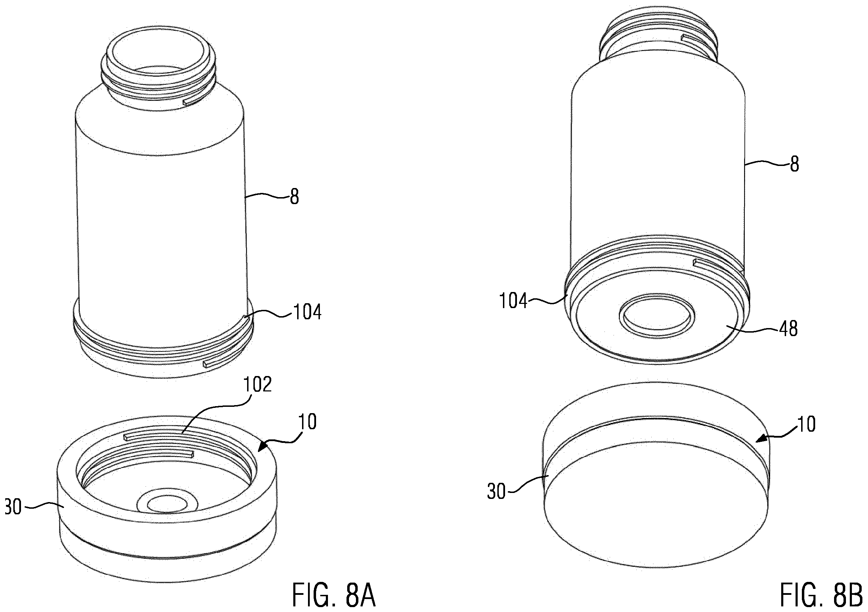

In den Figuren 7A, 7B ist ein drittes Ausführungsbeispiel mit einer Schraubverbindung dargestellt. Das Gehäuse 30 des Flaschenbodens 10 bildet nach diesem Ausführungsbeispiel ein Außengewinde 98 aus, das mit einem am Flaschenkörper 8 ausgebildeten Innengewinde 100 zusammenwirkt. Ein viertes Ausführungsbeispiel nach den Figuren 8A, 8B hat ebenfalls eine Schraubverbindung, wobei hier das Gehäuse 30 des Flaschenbodens 10 ein Innengewinde 102 ausbildet, das mit einem am Flaschenkörper 8 ausgebildeten Außengewinde 104 zusammenwirkt.

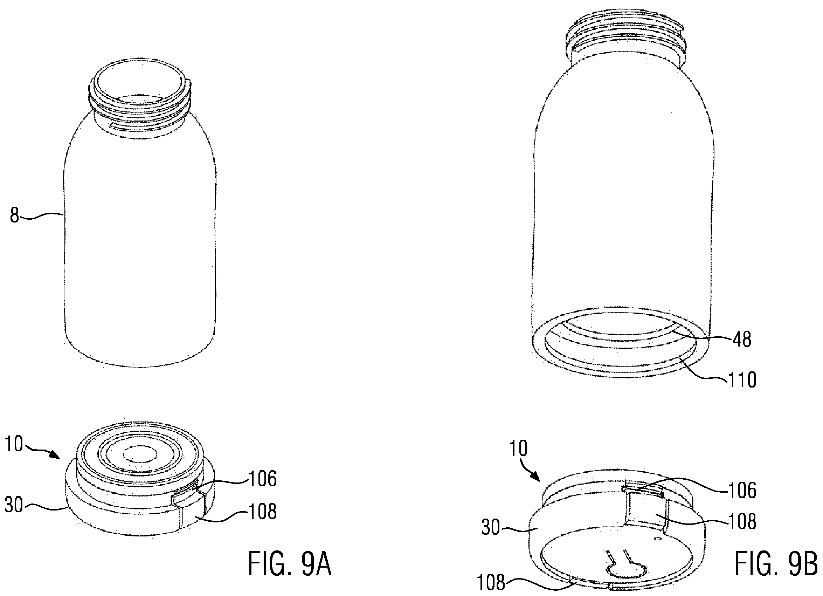

In den Figuren 9A, 9B ist ein fünftes Ausführungsbeispiel mit einem Schnappverschluss dargestellt. Nach diesem Ausführungsbeispiel weist der Flaschenboden 10 zwei gegenüberliegende Rastnasen 106 auf, die durch manuelle Betätigung eines Tastfelds 108 elastisch rückgehalten werden können, d.h. radial nach innen gedrückt werden können, um sie bei dem Einschieben des Flaschenbodens 10 in den Flaschenkörper 8 hinter einen am Flaschenkörper 8 ausgebildeten Rastvorsprung 110 zu verbringen (in der Figur ist lediglich eine Rastnase zu erkennen). Der Rastvorsprung 110 ist hierbei als umfänglich durchgängiger Steg an der Innenumfangsfläche des dem Flaschenboden 10 zugewandten Endes des Flaschenkörpers 8 ausgebildet.

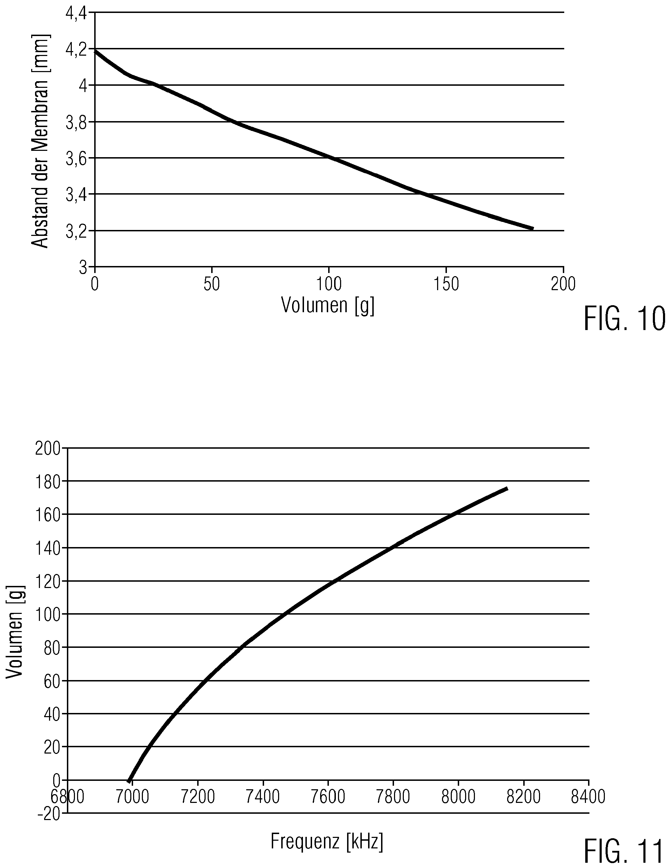

Die Figur 10 zeigt ein Schaubild, welches den Zusammenhang zwischen dem Milch-Füllvolumen in der Babyflasche (angegeben in Gramm) und dem Abstand des beweglichen Abschnitts 60 der Membran 28 von der Spule 74 der Sensorik 34 (angegeben in Millimeter) für ein Ausführungsbeispiel verdeutlicht. Aus dem Schaubild ist zu erkennen, dass es sich hierbei um einen im Wesentlichen linearen Zusammenhang handelt und dass der Abstand mit zunehmender Milch-Füllmenge abnimmt. Nach diesem Ausführungsbeispiel hat die Membran bei leerer Babyflasche einen Abstand von 4.2 mm von der Spule 74.

Die Figur 11 zeigt ein anderes Schaubild, welches den Zusammenhang zwischen dem Milch-Füllvolumen in der Babyflasche (angegeben in Gramm) und der Ausgangsfrequenz eines RLC-Schwingkreises (angegeben in Kilo-Herz) für ein Ausführungsbeispiel verdeutlicht. Dieser Zusammenhang wird durch verschiedene Parameter beeinflusst, die die Geometrie der Flasche betreffen, die Frequenz des RLC-Schwingkreises sowie Winkelangaben aufgrund von Signalen des Lagesensors 90, der in der Regel die Neigung der Flasche erfasst.

### Bezugszeichenliste

- 2: Vorrichtung
- 4: Konnektor
- 6: Babyflasche
- 8: Flaschenkörper
- 10: Flaschenboden
- 12: Trennelement
- 14: Brusthaube
- 16: Anschluss für Brustpumpe
- 18: Trichter
- 20: Flaschenhals
- 22: Flüssigkeitsreservoir
- 24: Außengewinde
- 26: Bajonettverschluss
- 28: elastische Membran
- 30: Gehäuse
- 32: Arretierarm
- 34: Sensorik
- 36: Gehäusedeckel
- 38: Distanzüberbrückungsbolzen
- 40: Befestigungslöcher im Gehäuse
- 42: Befestigungslöcher im Gehäusedeckel
- 44: Standfläche
- 46: Gehäuseflansch
- 48: ringförmiger Flansch
- 50: Oberseite der Membran
- 52: innerer bogenförmiger Ring
- 54: mittlerer Ring
- 56: äußerer Ring
- 58: Befestigungsabschnitt
- 60: beweglicher Abschnitt
- 62: randseitiger Flansch
- 64: Hinterschneidung
- 66: zweiter Flansch
- 68: Hinterschneidung
- 70: Kupferplatte
- 72: Leiterplatte
- 74: Spule
- 76: Loch
- 78: Durchgangslöcher
- 80: Kerbe
- 82a: Batterie
- 82b: Batterie
- 84: Ein/Aus-Schalter
- 86: Reset-Schalter
- 88: Mikroprozessor
- 90: Lagesensor
- 92: Hinterschneidung
- 94: Arretiersteg
- 98: Außengewinde
- 100: Innengewinde
- 102: Innengewinde
- 104: Außengewinde
- 106: Rastnase
- 108: Tastfeld
- 110: Rastvorsprung

## Patentansprüche

1. Vorrichtung (2), umfassend einen Konnektor (4), eine daran angekoppelte Babyflasche (6) mit einem Flaschenkörper (8) und einem lösbar am Flaschenkörper (8) befestigten Flaschenboden (10), und eine in dem Flaschenboden (10) angeordnete Sensorik (34) zur Bestimmung der Milch-Füllmenge in der Babyflasche (6), wobei die Vorrichtung ein flüssigkeits-undurchlässiges Trennelement umfasst, das in Richtung der Sensorik beweglich ist und ein Messelement aus elektrisch gut leitendem Material umfasst, **gekennzeichnet dadurch, dass** das Trennelement als eine elastische Membran mit einem zentrisch angeordneten metallischen Einsatz oder einer metallischen Beschichtung ausgebildet ist und dass die Sensorik eine Spule aufweist, die zentral im Flaschenboden und zu der Membran beabstandet angeordnet ist.

2. Vorrichtung (2) nach Anspruch 1, **dadurch gekennzeichnet, dass** die elastische Membran (28) bei aufrecht stehender Babyflasche (6) sich bei Zunahme der Milch-Füllmenge auf die Sensorik (34) hin und bei Abnahme der Milch-Füllmenge von der Sensorik (34) weg bewegt.

3. Vorrichtung (2) nach Anspruch 1 oder 2, **dadurch gekennzeichnet, dass** die Achse des metallischen Einsatzes (70) mit der Achse der Spule (74) zumindest angenähert fluchtet.

4. Vorrichtung (2) nach einem der vorherigen Ansprüche, **dadurch gekennzeichnet, dass** zwischen der Spule (74) und einer durch den Flaschenboden (10) ausgebildeten Standfläche (44) des lösbaren Flaschenbodens (10) elektronische Bauteile (82a, 82b, 84, 86, 88, 90) angeordnet sind.

5. Vorrichtung (2) nach einem der vorherigen Ansprüche, **dadurch gekennzeichnet, dass** der Flaschenboden (10) durch Verschrauben, einen Bajonettverschluss (26) oder einen Schnappverschluss an dem Flaschenkörper (8) der Babyflasche (6) befestigt ist.

6. Flasche (6) mit einem Flaschenkörper (8), einem lösbar am Flaschenkörper (8) befestigten Flaschenboden (10) und einer in dem Flaschenboden (10) angeordneten Sensorik (34) zur Bestimmung der Inhaltsmenge in der Flasche (6), wobei die Flasche ein flüssigkeits-undurchlässiges Trennelement umfasst, das in Richtung der Sensorik beweglich ist und ein Messelement aus elektrisch gut leitendem Material umfasst, **gekennzeichnet dadurch, dass** das Trennelement als eine elastische Membran mit einem zentrisch angeordneten metallischen Einsatz oder einer metallischen Beschichtung ausgebildet ist und dass die Sensorik eine Spule aufweist, die zentral im Flaschenboden und zu der Membran beabstandet angeordnet ist.

7. Flasche (6) nach Anspruch 6, **dadurch gekennzeichnet, dass** die elastische Membran (28) bei aufrecht stehender Flasche (6) sich bei Zunahme der Füllmenge auf die Sensorik (34) hin und bei Abnahme der Füllmenge von der Sensorik (34) weg bewegt.

8. Flasche (6) nach Anspruch 6 oder 7, **dadurch gekennzeichnet, dass** die Achse des metallischen Einsatzes (70) mit der Achse der Spule (74) zumindest angenähert fluchtet.

9. Flasche (6) nach einem der Ansprüche 6 bis 8, **dadurch gekennzeichnet, dass** zwischen der Spule (74) und einer durch den Flaschenboden (10) ausgebildeten Standfläche (44) des lösbaren Flaschenbodens (10) elektronische Bauteile (82a, 82b, 84, 86, 88, 90) angeordnet sind.

10. Flasche (6) nach einem der Ansprüche 6 bis 9, **dadurch gekennzeichnet, dass** der Flaschenboden (10) durch Verschrauben, einen Bajonettverschluss (26) oder einen Schnappverschluss an dem Flaschenkörper (8) der Flasche (6) befestigt ist.

## Claims

1. An apparatus (2) comprising a connector (4), a baby bottle (6) coupled thereto including a bottle body (8) and a bottle bottom (10) detachably attached to the bottle body (8) and sensor means (34) disposed in the bottle bottom (10) for determining the milk filling level in the baby bottle (6), wherein the apparatus comprises a liquid-impermeable separator element which is movable towards the sensor means and comprises a measuring element made of a material having good electrical conductivity , **characterized in that** the separator element is designed as an elastic membrane having a centrally arranged metallic insert or a metallic coating, and **in that** the sensor means includes a coil arranged centrally in the bottle bottom and spaced from the membrane.

2. The apparatus (2) according to claim 1, **characterized in that** with the baby bottle (6) standing upright, the elastic membrane (28) moves towards the sensor means (34) when the milk filling level increases and moves away from the sensor means (34) when the milk filling level decreases.

3. The apparatus (2) according to claims 1 or 2, **characterized in that** the axis of the metallic insert (70) is at least approximately in line with the axis of the coil (74).

4. The apparatus (2) according to any one of the preceding claims, **characterized in that** electronic components (82a, 82b, 84, 86, 88, 90) are arranged between the coil (74) and a supporting surface (44) of the detachable bottle bottom (10) defined by the bottle bottom (10).

5. The apparatus (2) according to any one of the preceding claims, **characterized in that** the bottle bottom (10) is attached to the bottle body (8) of the baby bottle (6) by means of screwing, a bayonet lock (26) or a snap lock.

6. A bottle (6) including a bottle body (8), a bottle bottom (10) detachably attached to the bottle body (8) and sensor means (34) arranged in the bottle bottom (10) for determining the amount of contents in the bottle (6), wherein the bottle comprises a liquid-impermeable separator element which is movable towards the sensor means and comprises a measuring element made of a material having good electrical conductivity, **characterized in that** the separator element is designed as an elastic membrane having a centrally arranged metallic insert or a metallic coating, and **in that** the sensor means includes a coil arranged centrally in the bottle bottom and spaced from the membrane.

7. A bottle (6) according to claim 6, **characterized in that** with the bottle (6) standing upright, the elastic membrane moves towards the sensor means (34) when the milk filling level increases and moves away from the sensor means (34) when the milk filling level decreases.

8. The bottle (2) according to claims 6 or 7, **characterized in that** the axis of the metallic insert (70) is at least approximately in line with the axis of the coil (74).

9. The bottle (6) according to one of claims 6 to 8, **characterized in that** electronic components (82a, 82b, 84, 86, 88, 90) are arranged between the coil (74) and a supporting surface (44) of the detachable bottle bottom (10) defined by the bottle bottom (10).

10. The bottle (6) according to one of claims 6 to 9, **characterized in that** the bottle bottom (10) is attached to the bottle body (8) of the bottle (6) by means of screwing, a bayonet lock (26) or a snap lock.

## Revendications

1. Dispositif (2), comprenant un raccord (4), un biberon (6) couplé à celui-ci présentant un corps de biberon (8) et un fond de biberon (10) fixé de manière amovible au corps de biberon (8), et un capteur (34) disposé dans le fond de biberon (10) pour déterminer la quantité de remplissage de lait dans le biberon (6), le dispositif comprenant un élément de séparation imperméable aux liquides, qui est mobile dans le sens du capteur et qui comprend un élément de mesure en un matériau hautement électriquement conducteur, **caractérisé en ce que** l'élément de séparation est conçu sous la forme d'une membrane élastique ayant un insert métallique ou un revêtement métallique disposé au centre et **en ce que** le capteur présente une bobine qui est disposée au centre dans le fond du biberon et à distance de la membrane.

2. Dispositif (2) selon la revendication 1, **caractérisé en ce que**, lorsque le biberon (6) se trouve en position verticale, la membrane élastique (28) se déplace vers le capteur (34) lorsque la quantité de remplissage de lait augmente et s'éloigne du capteur (34) lorsque la quantité de remplissage de lait diminue.

3. Dispositif (2) selon la revendication 1 ou 2, **caractérisé en ce que** l'axe de l'insert métallique (70) est au moins approximativement aligné sur l'axe de la bobine (74).

4. Dispositif (2) selon l'une des revendications précédentes, **caractérisé en ce que** des composants électroniques (82a, 82b, 84, 86, 88, 90) sont disposés entre la bobine (74) et une surface d'appui (44) du fond de bouteille amovible (10) formée par le fond de la bouteille (10).

5. Dispositif (2) selon l'une des revendications précédentes, **caractérisé en ce que** le fond (10) du biberon est fixé au corps (8) du biberon (6) par vissage, par une fermeture à baïonnette (26) ou par encliquetage.

6. Bouteille (6) ayant un corps de bouteille (8), un fond de bouteille (10) fixé de manière amovible au corps de bouteille (8) et un capteur (34) disposé dans le fond de bouteille (10) pour déterminer la quantité de remplissage dans la bouteille (6), la bouteille comprenant un élément de séparation imperméable aux liquides, qui est mobile dans le sens du capteur et qui comprend un élément de mesure en matériau hautement électriquement conducteur, **caractérisé en ce que** l'élément de séparation est conçu comme une membrane élastique ayant un insert ou un revêtement métallique disposé au centre et **en ce que** le capteur présente une bobine qui est disposée au centre dans le fond de la bouteille et à distance de la membrane.

7. Bouteille (6) selon la revendication 6, **caractérisée en ce que**, lorsque la bouteille (6) se trouve en position verticale, la membrane élastique (28) se déplace vers le capteur (34) lorsque la quantité de remplissage augmente et s'éloigne du capteur (34) lorsque la quantité de remplissage diminue.

8. Bouteille (6) selon la revendication 6 ou 7, **caractérisée en ce que** l'axe de l'insert métallique (70) est au moins approximativement aligné sur l'axe de la bobine (74).

9. Bouteille (6) selon l'une des revendications 6 à 8, **caractérisée en ce que** des composants électroniques (82a, 82b, 84, 86, 88, 90) sont disposés entre la bobine (74) et une surface d'appui (44) du fond de bouteille amovible(10), formée par le fond (10) de la bouteille.

10. Bouteille (6) selon l'une quelconque des revendications 6 à 9, **caractérisée en ce que** le fond (10) de la bouteille est fixé au corps (8) de la bouteille (6) par vissage, par une fermeture à baïonnette (26) ou par encliquetage.
